# EUROPEAN PATENT APPLICATION

(11) **EP 3 779 456 A1**
(43) Date of publication of application: **17.02.2021**
(21) Application number: 19774266.1
(22) Date of filing: 28.03.2019
(51) Int. Cl.: G01N 33/92, C12Q 1/26, C12Q 1/44

(54) **QUANTIFICATION METHOD, QUANTIFICATION REAGENT AND QUANTIFICATION KIT FOR LIPOPROTEIN CHOLESTEROL**

(30) Priority: 28.03.2018 JP 2018062143
(71) Applicant: Denka Company Limited, Tokyo 103-8338 (JP)
(72) Inventor: SATOH, Noriyuki, Gosen-shi, Niigata 959-1834 (JP); IKAIDA, Makoto, Gosen-shi, Niigata 959-1834 (JP); HIRAO, Yuhko, Gosen-shi, Niigata 959-1834 (JP)
(74) Representative: Mewburn Ellis LLP
(86) International application number: PCT/JP2019/013766
(87) International publication number: WO 2019/189642

(57) **Abstract**

Provided arc a method which enables more accurate quantification of lipoprotein cholesterol in a test sample, and a quantification reagent and a quantification kit used for this method. The present invention provides a quantification method for lipoprotein cholesterol in a test sample containing a lipoprotein optionally using a quantification reagent, the method including adding a phospholipid to the test sample or the quantification reagent. The present invention also provides a quantification reagent for cholesterol in a lipoprotein used in the method of the present invention, the quantification reagent containing a phospholipid. The present invention also provides a quantification kit for lipoprotein cholesterol used in the method of the present invention, the quantification kit containing a phospholipid.

## Description

### TECHNICAL FIELD

The present invention relates to a method, a reagent, and a kit for quantifying cholesterol in lipoprotein.

### BACKGROUND ART

Based on the difference in the density as determined by ultracentrifugation, lipoproteins contained in blood can be divided into chylomicron, very low-density lipoprotein (which may be hereinafter referred to as VLDL), intermediate-density lipoprotein (which may be hereinafter referred to as IDL), low-density lipoprotein (which may be hereinafter referred to as LDL), and high-density lipoprotein (which may be hereinafter referred to as HDL). In recent years, these lipoproteins are further divided depending on the density and size. For example, small and highly dense LDLs include small-particle low-density lipoprotein (which may be hereinafter referred to as small, dense LDL or sdLDL). These lipoproteins contain various amounts of lipids such as triglyceride and cholesterol; proteins; and the like; and are known to exhibit different actions in vivo.

Examples of currently known methods of measuring cholesterol in lipoproteins include ultracentrifugation and NMR. Ultracentrifugation is a method in which centrifugation is carried out to perform fractionation utilizing the difference in the density of lipoprotein. This method has drawbacks in that the operation requires a skill; the method takes many days; and the cost is high. NMR. which is a method wherein the number of particles of lipoprotein is measured by nuclear magnetic resonance, is not commonly employed since the method requires special equipment.

In recent years, the direct method is rapidly becoming common since the method does not require laborious operations, and since automatic analyzers can be used therefor. Examples of a method of measuring HDL include a method disclosed in Patent Document 1, in which non-HDL lipoproteins arc sufficiently reacted using, as a flocculant, cyclodextrin sulfate, and then an enzyme modified with polyethylene glycol is allowed to act to achieve specific measurement of cholesterol in HDL. Patent Document 2 discloses a method in which a surfactant inhibitory to reaction of non-HDL lipoproteins, and a surfactant which specifically dissolves HDL, are used. Patent Document 3 discloses a method comprising: a first step of eliminating non-HDL lipoproteins using catalase; and a second step of measuring HDL using a surfactant that specifically acts on HDL. Patent Document 4 discloses a method in which an antibody against non-HDL lipoprotein is allowed to act first, and then HDL is dissolved, followed by detecting cholesterol. Examples of a method of measuring small, dense LDL include a method disclosed in Patent Document 5, comprising: a first step of eliminating cholesterol of the lipoproteins other than small, dense LDL using a surfactant that acts on the lipoproteins other than small, dense LDL; and then quantifying cholesterol of the remaining small, dense LDL. Patent Document 6 discloses a method comprising: a first step of eliminating cholesterol of the LDLs other than small, dense LDL using sphingomyelinase; and then quantifying cholesterol in the remaining small, dense LDL. As a measurement method for triglyceride-rich lipoprotein (TRL) in which the total density of chylomicron (including chylomicron remnant), VLDL (including VLDL remnant), and IDL is less than 1.019 g/cm³, Patent Document 7 discloses a method in which cholesterol of the non-TRL lipoproteins is eliminated using a surfactant that acts on the non-TRL lipoproteins, and then cholesterol in the remaining TRL is quantified.

### PRIOR ART DOCUMENTS

### [Patent Documents]

[Patent Document 1] JP 8-131197 A
[Patent Document 2] JP 11-56395 A
[Patent Document 3] WO 98/26090
[Patent Document 4] JP 9-96637 A
[Patent Document 5] JP 2013-148589 A
[Patent Document 6] WO 09/048143
[Patent Document 7] JP 2017-209035 A

### SUMMARY OF THE INVENTION

### PROBLEMS TO BE SOLVED BY THE INVENTION

An object of the present invention is to provide a method which enables more accurate quantification of lipoprotein cholesterol in a test sample, and a quantification reagent and a quantification kit used for this method.

### MEANS FOR SOLVING THE PROBLEMS

The present inventors intensively studied to discover that, by adding a phospholipid to a test sample or a quantification reagent, improved linearity of measured values (linearity of correlation between the dilution level of the test sample and the reaction absorbance of cholesterol) can be achieved. The present inventors then assumed that more accurate measurement of cholesterol in a lipoprotein may be possible by utilization of this discovery, thereby completing the present invention.

More specifically, the present invention is as follows.
[1] A method for quantifying lipoprotein cholesterol in a test sample containing a lipoprotein optionally using a quantification reagent, the method comprising adding a phospholipid to the test sample or the quantification reagent.
[2] The method according to [1], wherein the phospholipid is phosphatidylcholine (PC) or lysophosphatidylcholine (LPC).
[3] The method according to [1], wherein the phospholipid is a phospholipid-like surfactant.
[4] The method according to [1], wherein the phospholipid is a lipoprotein which is different from the lipoprotein containing the cholesterol to be quantified.
[5] The method according to [4], wherein the phospholipid is high-density lipoprotein (IIDL).
[6] The method according to any one of [1] to [5], wherein the lipoprotein is triglyceride-rich lipoprotein (TRL).
[7] The method according to any one of [1] to [5], wherein the lipoprotein is small, dense LDL.
[8] The method according to any one of [1] to [7], wherein the lipoprotein cholesterol is quantified using a quantification reagent.
[9] A reagent for quantifying cholesterol in a lipoprotein, used in the method according to any one of [1] to [8], the quantification reagent comprising a phospholipid.
[10] Use of the reagent according to [9], as a reagent for quantifying lipoprotein cholesterol in a test sample containing a lipoprotein.
[11] A kit for quantifying lipoprotein cholesterol, used in the method according to any one of [1] to [8], the quantification kit comprising a phospholipid.
[12] Use of the kit according to [11], as a kit for quantifying lipoprotein cholesterol in a test sample containing a lipoprotein.

### EFFECT OF THE INVENTION

By the present invention, a novel method which enables more accurate quantification of lipoprotein cholesterol in a test sample, and a quantification reagent and a quantification kit used for this method, were provided.

### BRIEF DESCRIPTION OF THE DRAWINGS

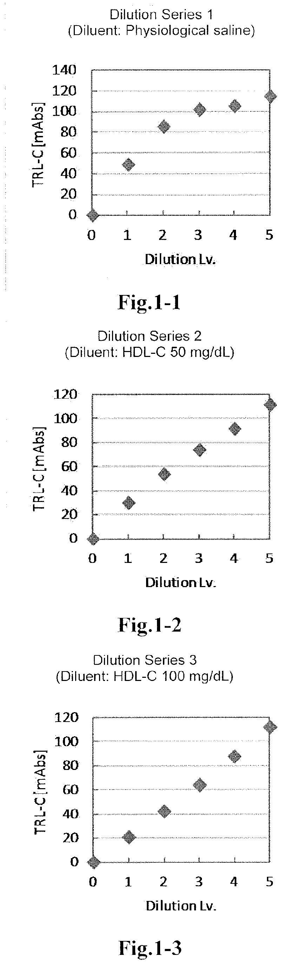

Fig. 1-1 is a diagram in Example 1, in which the reaction absorbance of TRL-C is plotted against the dilution level of Dilution Series 1 (human serum diluted with physiological saline).
Fig. 1-2 is a diagram in Example 1, in which the reaction absorbance of TRL-C is plotted against the dilution level of Dilution Series 2 (human serum diluted with Diluent A, which contains HDL).
Fig. 1-3 is a diagram in Example 1, in which the reaction absorbance of TRL-C is plotted against the dilution level of Dilution Series 3 (human serum diluted with Diluent B, which contains HDL).
Fig. 2-1 is a diagram in Example 2, in which the reaction absorbance of sdLDL-C is plotted against the dilution level of Dilution Series 4 (human serum diluted with physiological saline).
Fig. 2-2 is a diagram in Example 2, in which the reaction absorbance of sdLDL-C is plotted against the dilution level of Dilution Series 5 (human serum diluted with Diluent B, which contains HDL).
Fig. 3-1 is a diagram in Example 1, in which the reaction absorbance of TRL-C is plotted against the dilution level of Dilution Series 1 (human scrum diluted with physiological saline), which diagram was obtained in a case where TRL-C Reagent 1-1 (HDL-C: 0 mg/dL) was used.
Fig. 3-2 is a diagram in which the reaction absorbance of TRL-C is plotted against the dilution level of Dilution Series 1, which diagram was obtained in a case where TRL-C Reagent 1-2 (HDL-C: 2 mg/dL) in Example 3 was used.
Fig. 3-3 is a diagram in which the reaction absorbance of TRL-C is plotted against the dilution level of Dilution Series 1, which diagram was obtained in a case where TRL-C Reagent 1-3 (HDL-C: 3 mg/dL) in Example 4 was used.
Fig. 4-1 is a diagram in which the reaction absorbance of TRL-C is plotted against the dilution level of Dilution Series 1 (human serum diluted with physiological saline), which diagram was obtained in a case where TRL-C Reagent 1-1 (phospholipid: 0 mg/dL) in Example 1 was used.
Fig. 4-2 is a diagram in which the reaction absorbance of TRL-C is plotted against the dilution level of Dilution Series 1, which diagram was obtained in a case where TRL-C Reagent 1-4 (phospholipid PC: 400 mg/dL) in Example 5 was used.
Fig. 4-3 is a diagram in which the reaction absorbance of TRL-C is plotted against the dilution level of Dilution Series 1, which diagram was obtained in a case where TRL-C Reagent 1-5 (phospholipid PC: 800 mg/dL) in Example 6 was used.
Fig. 4-4 is a diagram in which the reaction absorbance of TRL-C is plotted against the dilution level of Dilution Series 1, which diagram was obtained in a case where TRL-C Reagent 1-6 (phospholipid PC: 1600 mg/dL) in Example 7 was used.

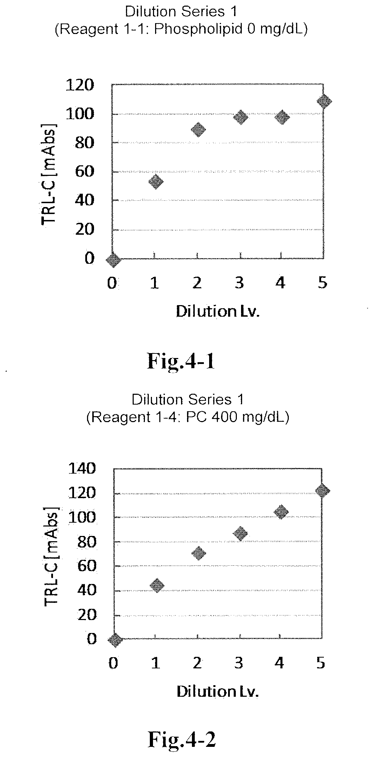

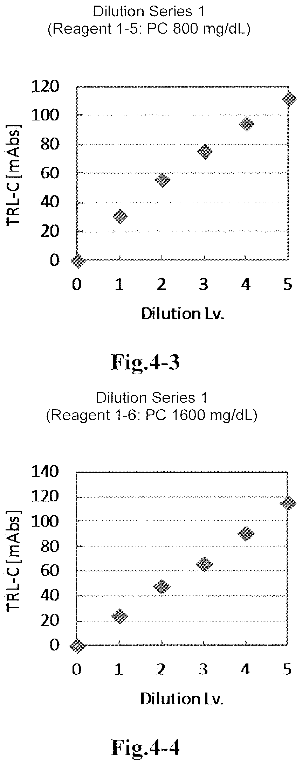

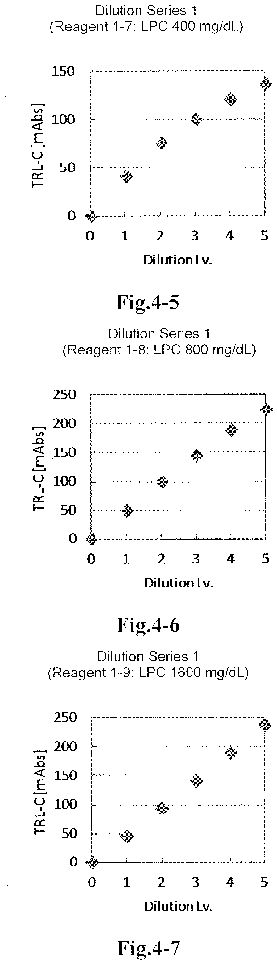

Fig. 4-5 is a diagram in which the reaction absorbance of TRL-C is plotted against the dilution level of Dilution Series 1, which diagram was obtained in a case where TRL-C Reagent 1-7 (phospholipid LPC: 400 mg/dL) in Example 8 was used.
Fig. 4-6 is a diagram in which the reaction absorbance of TRL-C is plotted against the dilution level of Dilution Series 1, which diagram was obtained in a case where TRL-C Reagent 1-8 (phospholipid LPC: 800 mg/dL) in Example 9 was used.
Fig. 4-7 is a diagram in which the reaction absorbance of TRL-C is plotted against the dilution level of Dilution Series 1, which diagram was obtained in a case where TRL-C Reagent 1-9 (phospholipid LPC: 1600 mg/dL) in Example 10 was used.

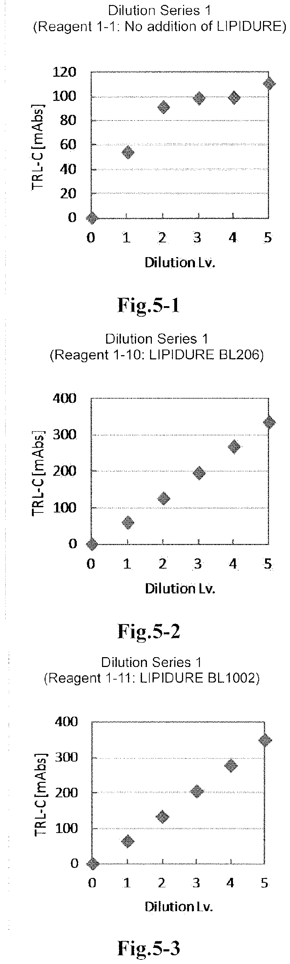

Fig. 5-1 is a diagram in which the reaction absorbance of TRL-C is plotted against the dilution level of Dilution Series 1 (human serum diluted with physiological saline), which diagram was obtained in a case where TRL-C Reagent 1-1 (without addition of LTPIDURE) in Example 1 was used.
Fig. 5-2 is a diagram in which the reaction absorbance of TRL-C is plotted against the dilution level of Dilution Series 1 (human serum diluted with physiological saline), which diagram was obtained in a case where TRL-C Reagent 1-10 (LIPIDURE BL206) in Example 11 was used.
Fig. 5-3 is a diagram in which the reaction absorbance of TRL-C is plotted against the dilution level of Dilution Series 1, which diagram was obtained in a case where TRL-C Reagent 1-11 (LIPIDURE BL1002) in Example 12 was used.

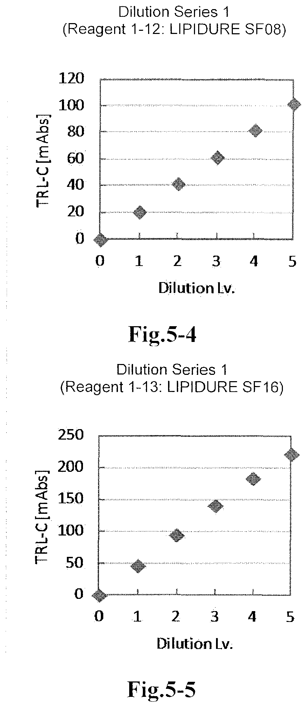

Fig. 5-4 is a diagram in which the reaction absorbance of TRL-C is plotted against the dilution level of Dilution Series 1, which diagram was obtained in a case where TRL-C Reagent 1-12 (LIPIDURE SF08) in Example 13 was used.
Fig. 5-5 is a diagram in which the reaction absorbance of TRL-C is plotted against the dilution level of Dilution Series 1, which diagram was obtained in a case where TRL-C Reagent 1-13 (LIPIDURE SF16) in Example 14 was used.

### MODE FOR CARRYING OUT THE INVENTION

The present invention is characterized in that, in quantification of cholesterol contained in a lipoprotein in a test sample, using as desired a quantification reagent, a phospholipid is added to the test sample or the quantification reagent.

Examples of the cholesterol contained in the lipoprotein include esterified cholesterol (cholesterol ester) and free cholesterol. When the term "cholesterol" is simply used in the present invention, it includes both of these.

The cholesterol in the lipoprotein to be quantified by the method of the present invention is cholesterol contained in spherical particles containing large amounts of triacylglycerol (triglyceride, neutral fat) and cholesterol indispensable for maintenance of the life of cells.

Lipoproteins containing cholesterol are classified based on electrophoresis or ultracentrifugation, and their examples include chylomicron, very low-density lipoprotein (VLDL), intermediate-density lipoprotein (IDL), low-density lipoprotein (LDL), small, dense LDL, and high-density lipoprotein (HDL). Examples of lipoproteins also include triglyceride-rich lipoprotein (TRL), which is composed of a combination of chylomicron (including chylomicron remnant), VLDL (including VLDL remnant), and IDL, and whose density is less than 1.019 g/cm³.

The test sample (specimen) to be subjected to the method of the present invention is not limited as long as it is for use in quantification of cholesterol in a lipoprotein. The test sample is usually a body fluid such as blood (including whole blood, serum, and plasma), or a dilution thereof.

As the method of quantifying lipoprotein cholesterol in the present invention, any well-known method in the art may be employed. Examples of the method include methods in which cholesterol oxidase and cholesterol esterase are allowed to act on cholesterol using a quantification reagent, and the resulting hydrogen peroxide is converted to a quinone pigment with peroxidase, a hydrogen donor, and a hydrogen acceptor, followed by quantifying the pigment by measurement of the absorbance. These are widely used, well-known enzymatic quantification methods, and described in, for example, WO 98/47005 and WO 98/26090. In the present invention, the cholesterol may be measured by any method as long as a phospholipid can be added to the test sample. Examples of methods without use of a quantification reagent include ultracentrifugation and NMR.

The quantification reagent for cholesterol used as desired in the present invention may be a reagent containing not only the later-mentioned phospholipid, but also, for example, cholesterol oxidase, cholesterol esterase, peroxidase, a hydrogen donor, and a hydrogen acceptor.

The phospholipid provided in the method of the present invention is a lipid containing a phosphate ester as a partial structure, and is mainly present as a major constituent of the cell membrane in the living body. Examples of the phospholipid in the present invention include glycerophospholipid, which has glycerol as a backbone, and sphingophospholipid, which has sphingosine as a backbone. Examples of the glycerophospholipid include phosphatidylcholine (PC), lysophosphatidylcholine (LPC), phosphatidylethanolamine, phosphatidylinositol, phosphatidylserine, and phosphatidylglycerol. Examples of the sphingophospholipid include sphingomyelin and sphingoethanolamine.

Examples of the phospholipid in the present invention also include phospholipid-like surfactants, and substances containing a phospholipid as a partial structure. The phospholipid-like surfactants are surfactants containing a phosphate ester as a partial structure. The phospholipid-like surfactants preferably have a structure containing a phosphorylcholine group (PC group), and their specific examples include LIPIDURE BL206 (NOF Corporation), LIPIDURE BL1002 (NOF Corporation), LIPIDURE SF08 (NOF Corporation), and LIPIDURE SF16 (NOF Corporation). The substances containing a phospholipid as a partial structure include: lipoproteins; liposomes; and biomembranes such as the cell membrane. Examples of the lipoproteins include chylomicron, very low-density lipoprotein (VLDL), intermediate-density lipoprotein (IDL), low-density lipoprotein (LDL), small, dense LDL, and high-density lipoprotein (HDL).

In cases where a lipoprotein is used as the phospholipid in the present invention, a lipoprotein which is different from the lipoprotein containing the cholesterol to be quantified by the method of the present invention is used. For example, in cases where cholesterol in triglyceride-rich lipoprotein (TRL) is to be quantified, a lipoprotein which is different from TRL, such as high-density lipoprotein (HDL), may be used.

In the present invention, a single kind of phospholipid may be used, or a plurality of kinds of phospholipids may be used in combination.

In the present invention, more than a certain amount of phospholipid may be present in the reaction liquid. The phospholipid is added to the test sample or the quantification reagent. The addition of the phospholipid to the test sample may be carried out by adding the phospholipid to a diluent to be used for, for example, diluting the test sample. Alternatively, the phospholipid may be preliminarily added to the quantification reagent so that the phospholipid is added to the test sample upon the measurement.

Regarding the amount of the phospholipid added, in cases where the phospholipid is added to the test sample, the phospholipid may be added such that its concentration in the diluent for diluting the test sample is within the range of usually 1 to 1000 mg/dL, preferably 10 to 500 mg/dL, more preferably 50 to 300 mg/dL.

In cases where the phospholipid is added to the quantification reagent, the amount of the phospholipid added varies depending on the type of the phospholipid. For example, when the phospholipid is glyccrophospholipid or sphingophospholipid, it may be added such that its concentration in the quantification reagent is within the range of usually 50 to 5000 mg/dL, preferably 200 to 3000 mg/dL, more preferably 500 to 2000 mg/dL. When the phospholipid is a lipoprotein, it may be added such that the concentration of lipoprotein cholesterol in the quantification reagent is within the range of usually 0.05 to 100 mg/dL, preferably 0.1 to 50 mg/dL, more preferably 0.5 to 10 mg/dL.

In the method of the present invention, the quantification reagent may be directly reacted with the test sample containing the lipoprotein, to quantify the cholesterol. However, more accurate quantification of the cholesterol in the lipoprotein to be quantified is possible by carrying out the method of the present invention by performing a first step (Step (1)) of selectively eliminating cholesterol in the lipoproteins other than the lipoprotein to be quantified in the test sample, and then performing a second step (Step (2)) of quantifying the cholesterol in the lipoprotein to be quantified remaining in the reaction system.

The term "reacting" with a lipoprotein as used in the present invention means that the structure of the lipoprotein is modified by a surfactant and/or an enzyme such that an enzyme can act more easily on the cholesterol therein.

In the Step (1) in the present invention, cholesterol in the lipoproteins other than the lipoprotein to be quantified is selectively eliminated. The term "eliminate" herein means that cholesterol is decomposed such that the decomposed product is not detected in the subsequent Step (2). Examples of the method of selectively eliminating cholesterol contained in the lipoproteins other than the lipoprotein to be quantified include a method in which cholesterol oxidase and cholesterol esterase are allowed to act on the test sample, and then the resulting hydrogen peroxide is removed. Examples of the method of removing the hydrogen peroxide include, but are not limited to, a method in which catalase is allowed to act on the hydrogen peroxide to decompose it into water and oxygen, and a method utilizing the action of peroxidase, in which, for example, a hydrogen donor compound that reacts with hydrogen peroxide to produce a colorless quinone is converted to the colorless quinone.

The method of selectively eliminating cholesterol in the particular lipoproteins in the first step is well known, and widely employed for quantification methods for LDL cholesterol (such as WO 98/47005), quantification methods for HDL cholesterol (such as WO 98/26090), and the like. The Step (1) in the present invention may also be carried out by these well-known methods.

The phospholipid in the present invention is preferably added in the Step (1) from the viewpoint of further improving the linearity of the measured values (linearity of correlation between the dilution level of the test sample and the reaction absorbance of cholesterol).

In the subsequent Step (2), the cholesterol in the lipoprotein to be quantified, remaining without being eliminated in the Step (1), is enzymatically quantified by the well-known method. In cases where cholesterol oxidase and cholesterol esterase are used in Step (1), the cholesterol oxidase and cholesterol esterase used in Step (1) may be used as they are in Step (2) without further addition.

In cases where the hydrogen peroxide produced in Step (1) is decomposed by catalase, and the catalase needs to be inhibited in Step (2), the catalase is inhibited using a catalase inhibitor such as sodium azide in Step (2).

At least one kind of surfactant may be used in the Step (1) and Step (2) in the present invention. Use of a suitable surfactant(s) promotes the enzyme reaction in each step.

The cholesterol oxidase that may be used in the present invention is not limited as long as it is an enzyme having an ability to produce hydrogen peroxide by oxidation of cholesterol. Examples of the cholesterol oxidase include animal- or microorganism-derived cholesterol oxidases. These may be products produced by genetic engineering, and/or may be chemically modified. The cholesterol esterase that may be used in the present invention is not limited as long as it acts on esterified cholesterol.

The amount of each of the cholesterol esterase and the cholesterol oxidase used in the present invention is not limited, and may be appropriately set. The amount is usually 0.001 U to 2000 U/mL, preferably 0.1 to 1000 U/mL.

The hydrogen donor used in the present invention is preferably an aniline derivative, and examples of the aniline derivative include N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), and N-(3-sulfopropyl)-3-methoxy-5-aniline (HMMPS).

As the hydrogen acceptor, 4-aminoantipyrinc, methylbenzothiazolone hydrazone, or the like may be used.

As the reaction liquid, various buffers used in ordinary biochemical reactions may be used, and the buffers preferably have a pH of 5 to 8. The solution is preferably Good's, Tris, phosphate, or glycine buffer solution, and is preferably bis(2-hydroxyethyl)iminotris(hydroxyethyl)methane (Bis-Tris), piperazine-1,4-bis(2-ethanesulfonic acid) (PIPES), piperazine-1,4-bis(2-ethanesulfonic acid). sesquisodium salt monohydrate (PIPES 1.5Na), 2-hydroxy-3-morpholinopropanesulfonic acid (MOPSO), N,N-bis(2-hydroxyethyl)-2-aminoethanesulfonic acid (BES), 2-[4-(2-hydroxyethyl)-1-piperazinyl]ethanesulfonic acid (HEPES), or piperazine-1,4-bis(2-hydroxy-3-propanesulfonic acid) (POPSO), which is a Good's buffer.

Each step in the present invention is preferably carried out at a pH of 5 to 10, more preferably at a pH of 6 to 8

The reaction temperature in each step is preferably 20°C to 45°C, more preferably 25°C to 40°C. The reaction time in each step is preferably 1 to 10 minutes, more preferably 3 to 7 minutes.

In the quantification method of the present invention, the reagents used may be divided into a plurality of reagent compositions. In the present invention, for example, a reagent composition (first reagent) for carrying out the step of eliminating cholesterol in the lipoproteins other than the lipoprotein to be quantified (that is, Step (1)), and a reagent composition (second reagent) for carrying out the step of measuring the cholesterol in the lipoprotein to be quantified (that is, Step (2)), may be prepared, and the combination of these two reagent compositions may be provided as a quantification kit for lipoprotein cholesterol.

The reagent composition for carrying out Step (1) contains a surfactant suitable for eliminating cholesterol in the lipoproteins other than the lipoprotein to be quantified. This reagent composition may further contain cholesterol oxidase; cholesterol esterase; a hydrogen donor such as an aniline derivative; catalase for eliminating hydrogen peroxide; or the like.

The reagent composition for carrying out Step (2) at least contains a surfactant that reacts with all lipoproteins described above. This reagent composition may further contain a hydrogen acceptor such as 4-aminoantipyrine; peroxidase; or the like.

The phospholipid in the present invention is preferably included in the reagent composition (first reagent) for carrying out Step (1), from the viewpoint of further improving the linearity of the measured values (linearity of correlation between the dilution level of the test sample and the reaction absorbance of cholesterol).

The content of the phospholipid in the reagent composition varies depending on the type of the phospholipid. When the phospholipid is glycerophospholipid or sphingophospholipid, the concentration of the phospholipid in the reagent composition is within the range of usually 50 to 5000 mg/dL, preferably 200 to 3000 mg/dL, more preferably 500 to 2000 mg/dL. When the phospholipid is a lipoprotein, the concentration of lipoprotein cholesterol in the reagent composition is within the range of usually 0.05 to 100 mg/dL, preferably 0.1 to 50 mg/dL, more preferably 0.5 to 10 mg/dL.

When necessary, the reagent composition for carrying out Step (1) and the reagent composition for carrying out Step (2) may contain a monovalent cation (such as a monovalent metal ion), a divalent cation (such as a divalent metal ion), or a salt thereof; a polyanion (such as heparin, dextran sulfate, or phosphotungstatc); or serum albumin. The pH of each reagent composition is near the neutral pH, for example, 5 to 9, preferably 6 to 8. The pH may be adjusted by addition of a buffer.

The cholesterol in the lipoprotein to be quantified by the method of the present invention may be quantified by adding the reagent composition for carrying out Step (1) to the test sample, allowing the reaction to proceed, adding the reagent composition for carrying out Step (2) to the resulting reaction product, allowing the reaction to proceed, and then measuring the absorbance.

The present invention is described below more concretely by way of Examples. However, the present invention is not limited to the following Examples.

### EXAMPLES

### Example 1

A commercially available HDL concentrate (manufactured by BRT) was diluted with physiological saline such that the HDL-C concentration became about 50 mg/dL, to prepare Diluent A; or diluted with physiological saline such that the IIDL-C concentration became about 100 mg/dL, to prepare Diluent B.

Human Serum 1 was diluted in 5 different levels with physiological saline according to the following Table 1, to prepare Dilution Series 1; diluted in 5 different levels with Diluent A according to the following Table 2, to prepare Dilution Series 2; or diluted in 5 different levels with Diluent B according to the following Table 3, to prepare Dilution Series 3.

**[Table 1]**

| (Dilution Series 1) | | | | | | |
|---|---|---|---|---|---|---|
| Dilution Lv. | 0 | 1 | 2 | 3 | 4 | 5 |
| Human serum 1 | 0 µL | 100 µL | 200 µL | 300 µL | 400 µL | 500 µL |
| Physiological saline | 500 µL | 400 µL | 300 µL | 200 µL | 100 µL | 0 µL |

**[Table 2]**

| (Dilution Series 2) | | | | | | |
|---|---|---|---|---|---|---|
| Dilution Lv. | 0 | 1 | 2 | 3 | 4 | 5 |
| Human serum 1 | 0 µL | 100 µL | 200 µL | 300 µL | 400 µL | 500 µL |
| Diluent A | 500 µL | 400 µL | 300 µL | 200 µL | 100 µL | 0 µL |

**[Table 3]**

| (Dilution Series 3) | | | | | | |
|---|---|---|---|---|---|---|
| Dilution Lv. | 0 | 1 | 2 | 3 | 4 | 5 |
| Human serum 1 | 0 µL | 100 µL | 200 µL | 300 µL | 400 µL | 500 µL |
| Diluent B | 500 µL | 400 µL | 300 µL | 200 µL | 100 µL | 0 µL |

A TRL-C measurement reagent composed of the following first reagent (TRL-C Reagent 1-1) and second reagent (TRL-C Reagent 2) was prepared.

### First reagent (TRL-C Reagent 1-1)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

The TRL-C measurement reagent was used for the test samples of Dilution Series 1, Dilution Series 2, and Dilution Series 3, and the reaction absorbances were measured using a Hitachi Autoanalyzer Type 7180 by the following method.

To 3 µL of each test sample, 150 µL of the first reagent was added, and the reaction was allowed to proceed at 37°C for 5 minutes. Subsequently, 50 µL of the second reagent was added thereto, and the reaction was allowed to proceed for 5 minutes, followed by measurement of the reaction absorbance at a primary wavelength of 600 nm and a secondary wavelength of 700 nm.

The results are shown in Fig. 1-1 to Fig. 1-3, which were drawn by taking the dilution level of each of Dilution Series 1, Dilution Series 2, and Dilution Series 3 along the x-axis, and the reaction absorbance of TRL-C along the y-axis.

As presented in Fig. 1, Dilution Series 2 and Dilution Series 3, which were prepared using Diluent A or Diluent B, which diluent contains HDL, exhibited better dilution linearity than Dilution Series 1, which was prepared using physiological saline.

### Example 2

Human Scrum 2 was diluted in 5 different levels with physiological saline according to the following Table 4, to prepare Dilution Series 4; or diluted in 5 different levels with Diluent B described in Example 1 according to the following Table 5, to prepare Dilution Series 5.

**[Table 4]**

| (Dilution Series 4) | | | | | | |
|---|---|---|---|---|---|---|
| Dilution Lv. | 0 | 1 | 2 | 3 | 4 | 5 |
| Human scrum 2 | 0 µl | 100 µL | 200 µL | 300 µL | 400 µL | 500 µL |
| Physiological saline | 500 µL | 400 µL | 300 µL | 200 µL | 100 µL | 0 µL |

**[Table 5]**

| (Dilution Series 5) | | | | | | |
|---|---|---|---|---|---|---|
| Dilution Lv. | 0 | 1 | 2 | 3 | 4 | 5 |
| Human serum 2 | 0 µL | 100 µL | 200 µL | 300 µL | 400 µL | 500 µL |
| Diluent B | 500 µL | 400 µL | 300 µL | 200 µL | 100 µL | 0 µL |

An sdLDL-C measurement reagent manufactured by Denka Seiken Co., Ltd. was used for the test samples of Dilution Series 4 and Dilution Series 5, and the reaction absorbances were measured using a Hitachi Autoanalyzer Type 7180 by the following method.

To 3 µL of each test sample, 150 µL of the first reagent was added, and the reaction was allowed to proceed at 37°C for 5 minutes. Subsequently, 50 µL of the second reagent was added thereto, and the reaction was allowed to proceed for 5 minutes, followed by measurement of the reaction absorbance at a primary wavelength of 600 nm and a secondary wavelength of 700 nm.

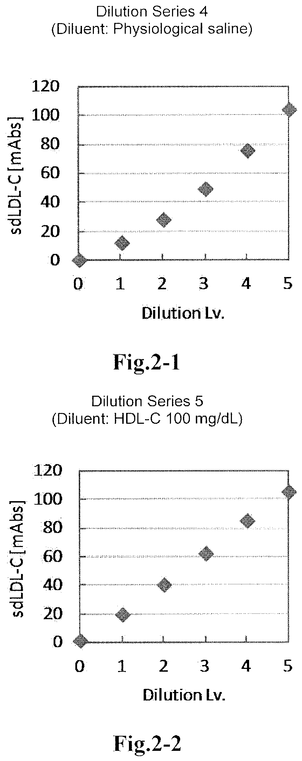

The results are shown in Fig. 2-1 and Fig. 2-2, which were drawn by taking the dilution level of each of Dilution Series 4 and Dilution Series 5 along the x-axis, and the reaction absorbance of sdLDL-C along the y-axis.

As presented in Fig. 2, Dilution Series 5, which was prepared using Diluent B, which diluent contains HDL, exhibited better dilution linearity than Dilution Series 4, which was prepared using physiological saline.

### Example 3

A TRL-C measurement reagent composed of the following first reagent (TRL-C Reagent 1-2) and second reagent (TRL-C Reagent 2) was prepared.

### First reagent (TRL-C Reagent 1-2)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether | [HLB: 12.8] 0.25% (w/v) |
| HDL concentrate | 2 mg/dL (HDL-C) |

### Second reagent (TRL-C Reagent 2)

| | |
|---|---|
| PIPES, p H6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 4

A TRL-C measurement reagent composed of the following first reagent (TRL-C Reagent 1-3) and second reagent (TRL-C Reagent 2) was prepared.

### First reagent (TRL-C Reagent 1-3)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenylether [HLB: 12.8] | 0.25% (w/v) |
| HDL concentrate | 3 mg/dL (HDL-C) |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

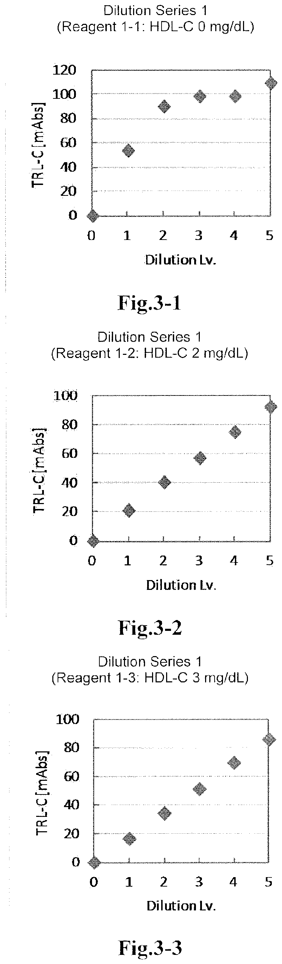

The TRL-C measurement reagents described in Examples 1, 3, and 4 were used for the test samples of Dilution Series 1 described in Example 1, and the reaction absorbances of TRL-C were measured by the same method as in Example 1. The results are shown in Fig. 3-1 to Fig. 3-3, which were drawn by taking the dilution level of the dilution series along the x-axis, and the reaction absorbance of TRL-C along the y-axis.

As presented in Fig. 3-1 to Fig. 3-3, better dilution linearity was found in the cases where the measurement was carried out using each TRL-C reagent containing HDL than in the cases where the measurement was carried out using the TRL-C reagent containing no HDL.

### Example 5

A TRL-C measurement reagent composed of the following first reagent (TRL-C Reagent 1-4) and second reagent (TRL-C Reagent 2) was prepared.

### First reagent (TRL-C Reagent 1-4)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid (phosphatidylcholine (PC)) | 400 mg/dL |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 6

A TRL-C measurement reagent composed of the following first reagent (TRL-C Reagent 1-5) and second reagent (TRL-C Reagent 2) was prepared.

### First reagent (TRL-C Reagent 1-5)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid (phosphatidylcholine (PC)) | 800 mg/dL |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 7

A TRL-C measurement reagent composed of the following first reagent (TRL-C Reagent 1-6) and second reagent (TRL-C Reagent 2) was prepared.

### First reagent (TRL-C Reagent 1-6)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid (phosphatidylcholine (PC)) | 1600 mg/dL |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 8

A TRL-C measurement reagent composed of the following first reagent (TRL-C Reagent 1-7) and second reagent (TRL-C Reagent 2) was prepared.

### First reagent (TRL-C Reagent 1-7)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid (lysophosphatidylcholine (LPC)) | 400 mg/dL |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 9

A TRL-C measurement reagent composed of the following first reagent (TRL-C Reagent 1-8) and second reagent (TRL-C Reagent 2) was prepared.

### First reagent (TRL-C Reagent 1-8)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB:12.8] | 0.25% (w/v) |
| Phospholipid (lysophosphatidylcholine (LPC)) | 800 mg/dL |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 10

A TRL-C measurement reagent composed of the following first reagent (TRL-C Reagent 1-9) and second reagent (TRL-C Reagent 2) was prepared.

### First reagent (TRL-C Reagent 1-9)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid ( lysophosphatidylcholine (LPC)) | 1600 mg/dL |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

The TRL-C measurement reagents described in Example 1 and Examples 5 to 10 were used for the test samples of Dilution Series 1 described in Example 1, and the reaction absorbances of TRL-C were measured by the same method as in Example 1. The results are shown in Fig. 4-1 to Fig. 4-7, which were drawn by taking the dilution level of the dilution series along the x-axis, and the reaction absorbance of TRL-C along the y-axis.

As presented in Fig. 4-1 to Fig. 4-7, better dilution linearity was found in the cases where the measurement was carried out using each TRL-C reagent containing a phospholipid than in the cases where the measurement was carried out using the TRL-C reagent containing no phospholipid.

### Example 11

A TRL-C measurement reagent composed of the following first reagent (TRL-C Reagent 1-10) and second reagent (TRL-C Reagent 2) was prepared.

### First reagent (TRL-C Reagent 1-10)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid-like surfactant (LIPIDURE BL206 (NOF Corporation)) | 0.25% (w/v) |

### Second reagent (TRL-C Reagent 2)

| | |
|---|---|
| PIPES, pH | 6.8 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 12

A TRL-C measurement reagent composed of the following first reagent (TRL-C Reagent 1-11) and second reagent (TRL-C Reagent 2) was prepared.

### First reagent (TRL-C Reagent 1-11)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid-like surfactant (LIPIDURE BL1002 (NOF Corporation)) | 0.25% (w/v) |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 13

A TRL-C measurement reagent composed of the following first reagent (TRL-C Reagent 1-12) and second reagent (TRL-C Reagent 2) was prepared.

### First reagent (TRL-C Reagent 1-12)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid-like surfactant (LIPIDURE SF08 (NOF Corporation)) | 0.25% (w/v) |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

### Example 14

A TRL-C measurement reagent composed of the following first reagent (TRL-C Reagent 1-13) and second reagent (TRL-C Reagent 2) was prepared.

### First reagent (TRL-C Reagent 1-13)

| | |
|---|---|
| PIPES, pH 6.8 | 50 mmol/L |
| Cholesterol esterase | 2 U/mL |
| Cholesterol oxidase | 2 U/mL |
| Catalase | 1200 U/mL |
| TOOS | 2.0 mmol/L |
| Polyoxyethylene distyrenated phenyl ether [HLB: 12.8] | 0.25% (w/v) |
| Phospholipid-like surfactant (LIPIDURE SF16 (NOF Corporation)) | 0.25% (w/v) |

### Second reagent (TRL-C Reagent 2)

| | |
|---|---|
| PIPES, pH 6.8 | 50 mmol/L |
| 4-Aminoantipyrine | 4.0 mmol/L |
| Peroxidase | 20 units/mL |
| Sodium azide | 0.05% (w/v) |
| Polyoxyethylene alkyl ether | 0.5% (w/v) |

The TRL-C measurement reagents described in Example 1 and Examples 11 to 14 were used for the test samples of Dilution Series 1 described in Example 1, and the reaction absorbances of TRL-C were measured by the same method as in Example 1. The results are shown in Fig. 5-1 to Fig. 5-5, which were drawn by taking the dilution level of the dilution series along the x-axis, and the reaction absorbance of TRL-C along the y-axis.

As presented in Fig. 5-1 to Fig. 5-5. better dilution linearity was found in the cases where the measurement was carried out using each TRL-C reagent containing a phospholipid-like surfactant than in the cases where the measurement was carried out using the TRL-C reagent containing no phospholipid-like surfactant.

## Claims

1. A method for quantifying lipoprotein cholesterol in a test sample containing a lipoprotein optionally using a quantification reagent, the method comprising adding a phospholipid to the test sample or the quantification reagent.

2. The method according to claim 1, wherein the phospholipid is phosphatidylcholine (PC) or lysophosphatidylcholine (LPC).

3. The method according to claim 1, wherein the phospholipid is a phospholipid-like surfactant.

4. The method according to claim 1, wherein the phospholipid is a lipoprotein which is different from the lipoprotein containing the cholesterol to be quantified.

5. The method according to claim 4, wherein the phospholipid is high-density lipoprotein (HDL).

6. The method according to any one of claims 1 to 5, wherein the lipoprotein is triglyceride-rich lipoprotein (TRL).

7. The method according to any one of claims 1 to 5, wherein the lipoprotein is small, dense LDL.

8. The method according to any one of claims 1 to 7, wherein the lipoprotein cholesterol is quantified using a quantification reagent.

9. A reagent for quantifying cholesterol in a lipoprotein, used in the method according to any one of claims 1 to 8, the quantification reagent comprising a phospholipid.

10. Use of the reagent according to claim 9, as a reagent for quantifying lipoprotein cholesterol in a test sample containing a lipoprotein.

11. A kit for quantifying lipoprotein cholesterol, used in the method according to any one of claims 1 to 8, the quantification kit comprising a phospholipid.

12. Use of the kit according to claim 11, as a kit for quantifying lipoprotein cholesterol in a test sample containing a lipoprotein.
